# EUROPÄISCHE PATENTSCHRIFT

(11) **EP 0 591 763 B1**
(45) Veröffentlichungstag und Bekanntmachung des Hinweises auf die Patenterteilung: **29.07.1998**
(21) Anmeldenummer: 93115260.7
(22) Anmeldetag: 22.09.1993
(51) Int. Cl.: C07D 333/34, C07D 307/64, C07D 307/18, C07C 323/22, A23L 1/226

(54) **Verwendung von Thioalkandionen als Aromastoffe**
Use of thioalkanediones as flavouring agents
Utilisation des thioalkanediones comme des substances aromatisantes

(30) Priorität: 05.10.1992 DE 4233350
(43) Veröffentlichungstag der Anmeldung: 13.04.1994
(73) Patentinhaber: HAARMANN & REIMER GMBH, D-37601 Holzminden (DE)
(72) Erfinder: Bertram, Heinz-Jürgen, Dr., D-37603 Holzminden (DE); Emberger, Roland, Dr., D-37603 Holzminden (DE); Güntert, Matthias, Dr., D-37603 Holzminden (DE); Werkhoff, Peter, Dr., D-37671 Höxter (DE)
(74) Vertreter: Petrovicki, Wolfgang, Dr.

(56) Entgegenhaltungen:
- EP-A- 0 459 198
- JOURNAL OF CHEMICAL RESEARCH, SYNOPSES, Nr. 11 , 1988 Letchworth, GB, Seiten 356 - 357 J. TASUGI, ET AL.: 'A facile one-pot synthesis of vicinal di- and triketones from alpha-methylene ketones by N-bromosuccinimide-dimethyl sulphoxide oxidation'
- SYNTHESIS, Nr. 6 , Juni 1991 Stuttgart, DE, Seiten 487 - 490 K. MORI, ET AL.: 'Preparative bio-organic chemistry; XIV. Preparation of (2S,3S)-1-phenylthio-2,3-pentanediol and (2S,3S)-1-phenylsulphonyl-2,3-pentanediol by yeast reduction of the corresponding diketones, and conversion of the former to (+)-endo-brevicomin'
- CHEMISTRY LETTERS, Nr. 11 , 1987 Tokyo. JP, Seiten 2227 - 2228 T. FUJISAWA, ET AL.: '(2S,3S)-1-Phenylthio-2,3-butanediol. A useful chiral building block for a simple synthesis of (4R,5S)-5-hydroxy- hexan-4-olide and (4R,5S)-5-hydroxy- 2-hexen-4-olide'
- JOURNAL OF THE AMERICAN CHEMICAL SOCIETY, Bd. 104, Nr. 9 , 2. Mai 1982 Washington, DC, US, Seiten 2655 - 2657 J.W. KOZARICH, ET AL.: '(Glutathiomethyl)gyloxal: mirror-image catalysis by glyoxalase I'

## Beschreibung

Die Erfindung betrifft die Verwendung von Verbindungen der Formel 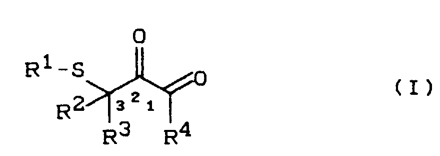 worin
- R¹: C₁-C₁₂-Alkyl, C₂-C₁₂-Alkenyl, C₂-C₁₂-Alkinyl, C₃-C₁₂-Cycloalkyl, C₆-C₁₂-Ar-C₁-C₆-alkyl, vorzugsweise Benzyl, gegebenenfalls substituiertes C₆-C₁₂-Aryl, vorzugsweise Phenyl, einen 5-oder 6-gliedrigen Heterocyclus mit Sauerstoff, Schwefel und/oder Stickstoff als Heteroatom und mit 0 bis 3 Doppelbindungen, der durch 1 bis 3 C₁-C₃-Alkylgruppen substituiert sein kann, oder Heterocyclo-methyl,
- R²: Wasserstoff oder C₁-C₄-Alkyl,
- R³, R⁴: unabhängig voneinander Wasserstoff, C₁-C₈-Alkyl, C₂-C₈-Alkenyl, C₂-C₈-Alkinyl, C₅-C₁₂-Cycloalkyl, C₆-C₁₂-Aryl, vorzugsweise Phenyl, oder
- R² und R³: gemeinsam oder
- R³ und R⁴: gemeinsam einen zweiwertigen Rest, der mit dem C-Atom 3 beziehungsweise mit den C-Atomen 1, 2 und 3 einen 5- bis 7-gliedrigen, vorzugsweise einen 5- bis 6-gliedrigen Ring enthaltend 0 bis 2 Heteroatome aus der Reihe Stickstoff, Sauerstoff, Schwefel bildet,
bedeuten.

Der Begriff "Alkyl" steht für geradkettiges oder verzweigtes Alkyl und umfaßt beispielsweise Methyl, Ethyl, n- und i-Propyl, n-, sek.-, i- und tert.-Butyl, n-, i- und tert.-Pentyl, n-Hexyl, i-Octyl, i-Nonyl, n-Decyl und n-Dodecyl.

Der Begriff "Alkenyl" steht für ein geradkettiges oder verzweigtes Alkenyl und umfaßt beispielsweise Vinyl und Allyl.

Der Begriff "Alkinyl" steht für geradkettiges oder verzweigtes Alkinyl und umfaßt beispielsweise Ethinyl und Propargyl.

Der Begriff "Cycloalkyl" umfaßt beispielsweise Cyclopropyl, Cyclobutyl, Cyclopentyl, Cyclohexyl und Cycloheptyl.

"Aralkyl" enthält vorzugsweise 1 bis 6, insbesondere 1 bis 4 C-Atome im geradkettigen oder verzweigten Alkylteil und vorzugsweise 6 bis 12 C-Atome, insbesondere Phenyl oder Naphthyl als Arylteil. Beispiele für solche Aralkylgruppen umfassen Benzyl, 2-Phenethyl, α- und β- Naphthylmethyl.

Der Begriff "Aryl" umfaßt beispielsweise Phenyl oder Naphthyl, insbesondere Phenyl.

Für substituierte Arylreste geeignete Substituenten umfassen beispielsweise Halogen, Cyano, Nitro, Hydroxy, Amino, Mercapto, geradkettiges oder verzweigtes Halogenalkyl mit 1 bis 6 Kohlenstoffatomen und 1 bis 13 gleichen oder verschiedenen Halogenatomen, jeweils geradkettiges oder verzweigtes Halogenalkylthio oder Halogenalkoxy mit jeweils 1 bis 6 Kohlenstoffatomen und jeweils 1 bis 13 gleichen oder verschiedenen Halogenatomen, jeweils geradkettiges oder verzweigtes Alkenyl oder Alkinyl mit 2 bis 6 Kohlenstoffatomen, jeweils geradkettiges oder verzweigtes Alkenyloxy oder Alkinyloxy mit 2 bis 6 Kohlenstoffatomen, Cycloalkyl mit 3 bis 8 Kohlenstoffatomen, Phenyl, Phenoxy, Phenylthio, Phenylalkylthio oder Phenylalkyloxy mit jeweils 1 bis 4 Kohlenstoffatomen im geradkettigen oder verzweigten Alkylteil.

Als heterocyclische Reste R¹ kommen vorzugsweise Furyl, Di- und Tetrahydrofuryl, Thienyl, Di- und Tetrahydrothienyl in Frage, die durch bis zu 2 C₁-C₃-Alkylgruppen substituiert sein können.

Unter dem Begriff "Heterocyclo-methyl" werden solche Reste verstanden, die aus einem (oben definierten) heterocyclischen Rest und einer Methylengruppe -CH₂- zusammengesetzt sind. In diesem Sinne bedeutet also "Pyrrylmethyl" den unten unter Nummer 19 aufgeführten R¹-Rest.

Wenn R² und R³ gemeinsam einen zweiwertigen Rest bilden, kommen vorzugsweise 1,4-Butylen und 1,5-Pentylen in Frage.

Wenn R³ und R⁴ gemeinsam einen zweiwertigen Rest bilden, kommen vorzugsweise 1,2-Ethylen und 1,3-Propylen in Frage, wobei diese Reste durch 1 oder 2 Methylgruppen substituiert sein können.

Bevorzugte Verbindungen der Formel (I) werden nachfolgend tabellarisch aufgezählt:

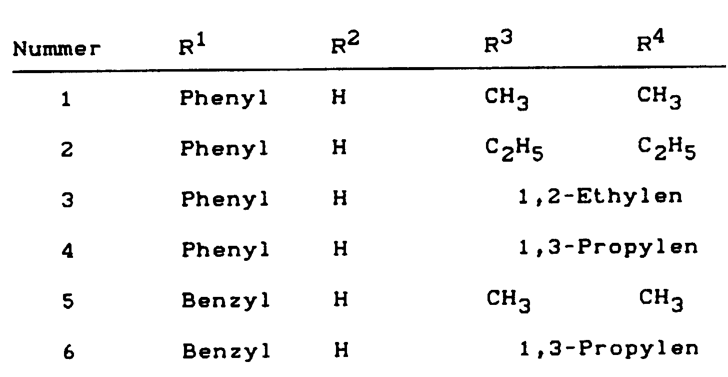

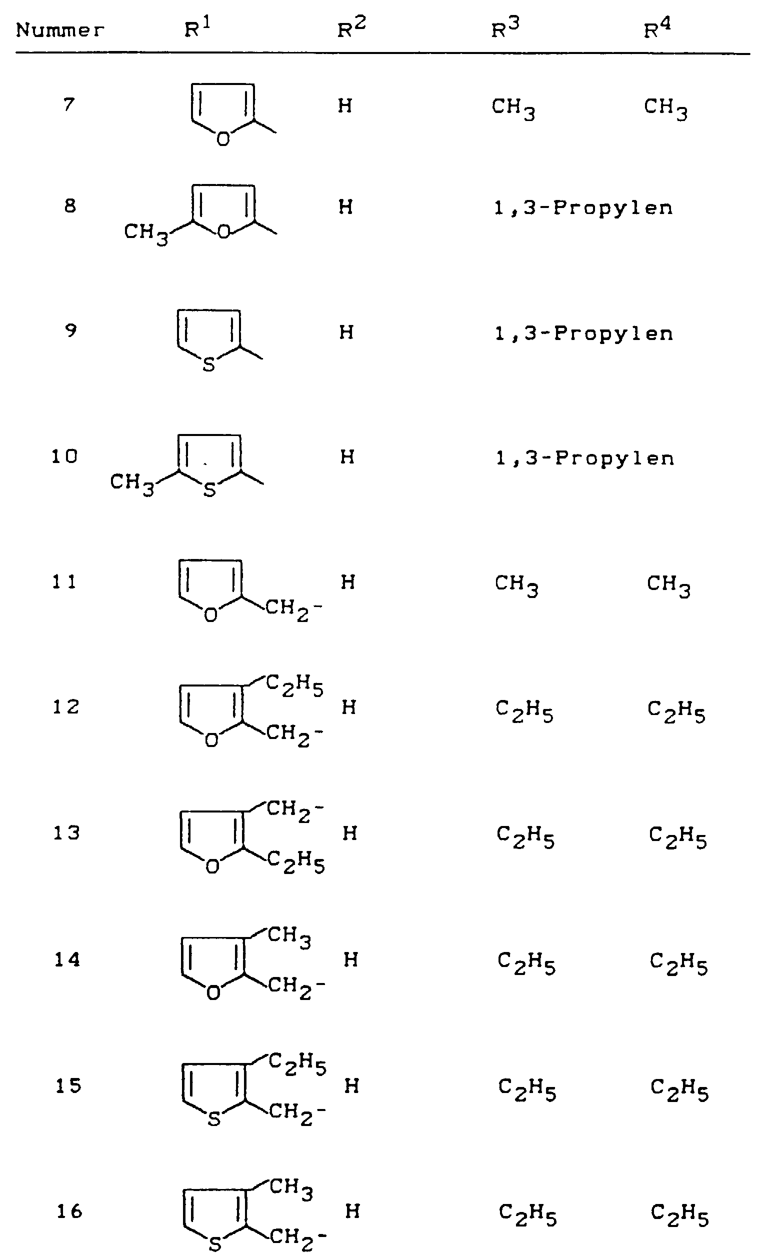

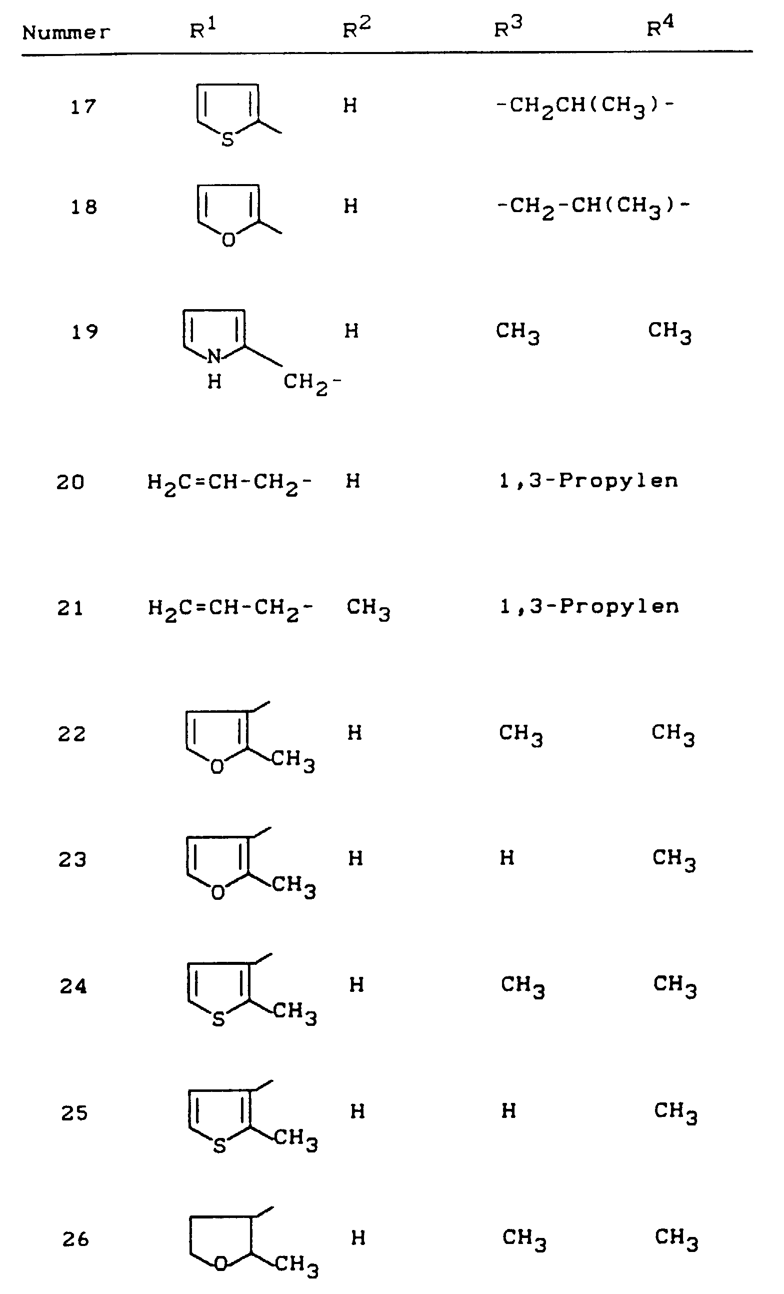

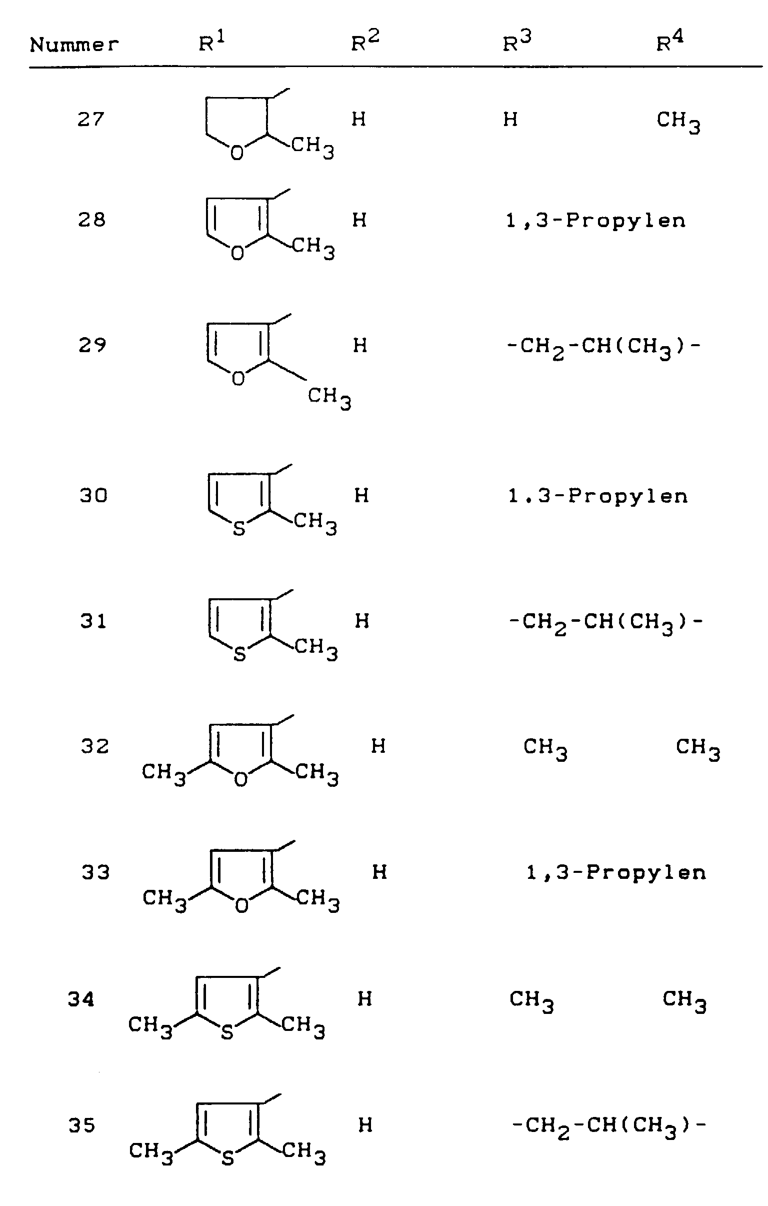

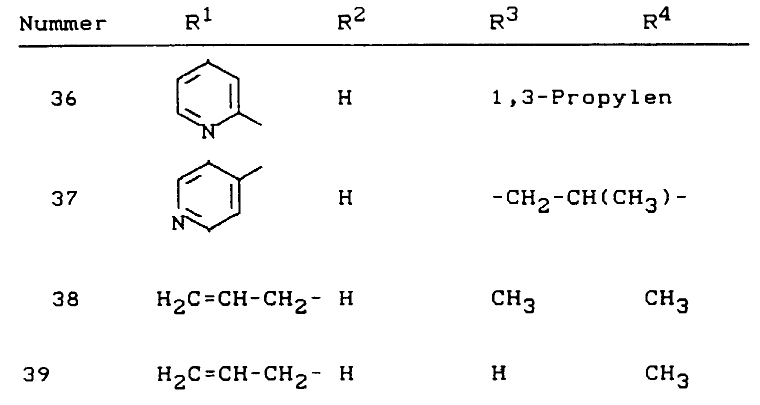

Die Verbindungen I sind bis auf wenige Ausnahmen neu. Die Ausnahmen sind 1-Phenylthio-2,3-pentandion (K. Mori et al., Synthesis 487 (1991)), 1-Phenylthio-2,3-butandion (T. Fujisawa et al., Chem. Lett. 2227 (1987)), sowie Ethylthiomethylglyoxal und (2-Hydroxyethyl)-methylglyoxal (J.W. Kozarich et al., J. Amer. Chem. Soc., 104, 2655 (1982)); die Verbindungen werden als Ausgangsstoffe für enzymatische Reduktionen eingesetzt.

Weiterer Gegenstand der Erfindung sind also Verbindungen der Formel (I), wobei die 4 bekannten Verbindungen ausgenommen sind.

Die Verbindungen (I) können beispielsweise hergestellt werden durch
a) Umsetzung von Thiolen der Formel

   R¹-SH (II)

   mit Ketonen der Formel 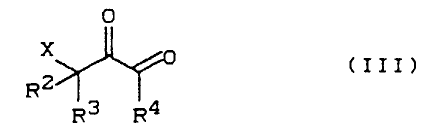 worin
   X ein Halogenatom, p-Toluolsulfonat, Methansulfonat, Acetyl oder Benzoyl bezeichnet,
b) Umsetzung von Methylthioethern der Formel

   R¹-S-CH₃ (IV)

   mit den Acetalen von α-Ketocarbonsäureestern der Formel 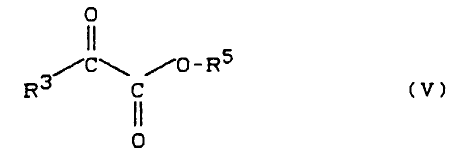 worin R⁵ für Methyl, Ethyl, n-Propyl oder Isopropyl steht,
   in Gegenwart starker Basen, z.B. Butyllithium (zur Deprotonierung) und anschließende Acetalspaltung (Synthesis 487 (1991)) oder
c) Umsetzung von Thioalkandionen der Formel 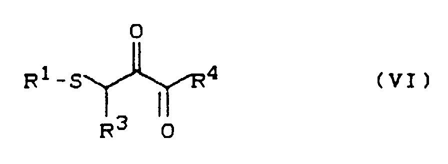 mit Verbindungen der Formel

   R²-X (VII)

   worin X die unter a) angegebene Bedeutung besitzt, in Gegenwart von Deprotonierungsmitteln,
   wobei die Substituenten R¹ bis R⁴ jeweils die für die Verbindungen (I) angegebenen Bedeutungen besitzen mit der Ausnahme, daß R¹ in Formel (IV) nicht für Wasserstoff steht.

Die Ausgangsverbindungen für die Herstellung der Verbindungen (I) sind bekannt oder können analog bekannten Verfahren hergestellt werden.

Die Verfahren zur Herstellung der Verbindungen (I) können ohne oder mit Verdünnungsmitteln durchgeführt werden. Als solche eignen sich inerte organische Lösungsmittel, wie z.B. insbesondere aliphatische, alicyclische oder aromatische Kohlenwasserstoffe, wie beispielsweise Benzol, Toluol, Xylol, Petrolether, Hexan, Cyclohexan, Ether wie Diethylether, Dioxan, Tetrahydrofuran oder Ethylenglykoldimethyl- oder -diethylether, Ketone wie Aceton oder Butanon, Nitrile wie Acetonitril oder Propionitril, Amide wie Dimethylformamid, Dimethylacetamid, N-Methylformanilid, Ester wie Essigsäureethylester oder Sulfoxide wie Dimethylsulfoxid oder Sulfone wie Sulfolan, Alkohole wie Methanol, Ethanol oder Isopropanol, chlorierte Kohlenwasserstoffe wie Chloroform oder Dichlormethan.

Die Verfahren zur Herstellung der Verbindungen (I) werden vorzugsweise in Gegenwart von Basen durchgeführt. Als solche eignen sich beispielsweise Alkalimetallhydride, -hydroxide, -amide, -alkoholate, -carbonate oder -hydrogencarbonate wie beispielsweise Lithiumdiisopropylamid (LDA), Natriumhydrid, Natriumamid, Natriumhydroxid, Natriummethylat, Natriumethylat, Kalium-t-butylat, Natriumcarbonat oder Natriumhydrogencarbonat oder auch tertiäre Amine wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU), Oxide von Erdalkalimetallen wie Magnesiumoxid oder Calciumoxid. Die Basen können, bezogen auf eingesetztes Thiol II oder eingesetzten Methylthioether IV, in äquimolarer Menge oder im Überschuß eingesetzt werden.

Die Temperaturen für die Verfahren a) bis c) können innerhalb weiter Bereiche variiert werden. Im allgemeinen arbeitet man bei Temperaturen zwischen -100°C und +100°C, vorzugsweise zwischen -78°C und +80°C, ganz besonders bevorzugt zwischen -78°C und +50°C.

Die Aufarbeitung der Reaktionsmischungen mit der Isolierung der Verbindungen (I) kann nach üblichen Methoden erfolgen.

Weiterer Gegenstand der Erfindung sind also Verfahren zur Herstellung der neuen Verbindungen (I).

Die Verbindungen (I) sind wertvolle Aromastoffe; sie zeichnen sich durch sehr niedrige Geschmacksschwellenwerte aus. So fand ein Testpanel aus 20 Prüfern in einem Dreieckstest bei der Anwendung von 4-(2-Methyl-3-furyl-thio)-pentan-2,3-dion in 0,5 gew.-%iger wässriger Kochsalzlösung bereits einen signifikanten Unterschied zwischen der Null-Probe und der nur 5 ppb 4-(2-Methyl-3-furyl-thio)-pentan-2,3-dion enthaltenden Kochsalzlösung. Für 4-(2-Methyl-3-furyl-thio)-butan-2,3-dion fand ein Testpanel aus 6 speziell geschulten Prüfern einen signifikanten Unterschied zwischen der 0,5 gew.-%igen Kochsalzlösung und der 5 ppb 4-(2-Methyl-3-tetrahydrofurylthio)-butan-2,3-dion enthaltenden 0,5 gew.-%igen Kochsalzlösung.

Die Geschmacksbeschreibungen für einige der erfindungsgemaßen Verbindungen der Formel (I) bei ihrer Anwendung in 0,5 gew.-%iger wässriger Kochsalzlösung lauten:
4-(2-Thienyl-thio)-pentan-2,3-dion:
   bei einem Zusatz von 8 ppb: Zwiebel, Knoblauch, Kohl
4-(2,5-Dimethyl-3-thienylthio)-butan-2,3-dion:
   bei einem Zusatz von 1 ppb: Pfirsichhaut, fruchtig, fleischig
4-(2,5-Dimethyl-3-thienylthio)-pentan-2,3-dion:
   bei einem Zusatz von 4 ppb: Brotrinde, Röstnote, Senfnote
4-(2-Methyl-3-furyl-thio)-pentan-2,3-dion:
   bei einem Zusatz von 0,8 ppm: Bratennote, Brühe, Hefecharakter, fleischig
4-(2-Methyl-3-furyl-thio)-butan-2,3-dion:
   bei einem Zusatz von 75 ppb: fleischig, Hydrolysat
4-(2-Methyl-3-thienyl-thio)-pentan-2,3-dion:
   bei einem Zusatz von 8 ppb: fleischig, Bratennote
4-(2-Methyl-3-thienyl-thio)-butan-2,3-dion:
   bei einem Zusatz von 8 ppb: Rinderbrühe, fleischig, Hefecharakter, schweflig
cis/trans-4-(2-Methyl-3-tetrahydrofurylthio)-pentan-2,3-dion:
   bei einem Zusatz von 0,5 ppm: Bratennote, fleischig, Brühe

Die Prozentangaben in den nachfolgenden Beispielen beziehen sich, sofern nichts anderes angegeben ist, auf das Gewicht.

### Beispiele

### Beispiel 1

1,12 g Kalium-tert.-butylat werden in 10 ml trockenem Tetrahydrofuran suspendiert. Bei -20°C wird unter Schutzgasatmosphare 1 ml 2-Methyl-3-furanthiol zugetropft, Bei dieser Temperatur wird für 30 Minuten nachgerührt. Dann werden bei -20°C 1,64 g 1-Brombutan-2,3-dion zugetropft. Man läßt den Ansatz auf Raumtemperatur kommen und rührt 2 Stunden nach. Dann werden 20 ml Diethylether zugegeben, anschließend wird auf 20 ml Wasser gegossen und die organische Phase abgetrennt. Die wässrige Phase wird dreimal mit je 10 ml Ether extrahiert, die vereinigten organischen Phasen werden mit Wasser gewaschen und dann über Natriumsulfat getrocknet.

Nach dem Abziehen des Lösungsmittels verbleiben 2,0 g eines hellgelben Öls als Rohprodukt.

Der Rückstand wird durch präparative Hochdruckflüssigkeitschromatographie (HPLC; Lösungsmittel: n-Pentan) gereinigt. Es werden 1,1 g 4-(2-Methyl-3-furanyl-thio)-butan-2,3-dion (Reinheitsgrad 93 %) erhalten. IR-, NMR- und Massenspektren der Verbindung stimmen mit der für sie angegebenen Struktur überein.

### Beispiel 2

Unter Schutzgasatmosphäre werden 3 ml trockenes Tetrahydrofuran und 0,70 ml Diisopropylamin vorgelegt. Dann wird auf 0°C abgekühlt und es werden 2,66 ml einer 1,6 molaren Butyllithium-Lösung in Hexan zugetropft. Man laßt 10 Minuten bei 0°C nachrühren, danach wird auf -60°C abgekühlt. Bei dieser Temperatur werden sehr langsam 0,99 g 4-(2-Methyl-3-furylthio)-butan-2,3-dion, gelöst in 5 ml trockenem Tetrahydrofuran, zugetropft. Nach beendeter Zugabe wird für 30 Minuten bei -60°C nachgerührt; die Farbe der Lösung ändert sich dabei von gelb zu braun. Dann werden bei -60°C 0,312 ml Methyljodid zugetropft. Es wird anschließend für 30 Minuten bei -60°C und für 2 Stunden bei Raumtemperatur nachgerührt. Der Ansatz wird auf Wasser gegossen und mit Diethylether extrahiert. Die wässrige Phase wird dreimal mit Ether extrahiert, anschließend werden die vereinigten organischen Phasen mit wäßriger Natriumhydrogencarbonatlösung gewaschen und über Natriumsulfat getrocknet.

Nach dem Abziehen des Lösungsmittels verbleiben 0,9 g eines braunen Öls als Rohprodukt.

Der Rückstand wird durch HPLC (Lösungsmittel: n-Pentan) gereinigt. Es wird 0,1 g 4-(2-Methyl-furylthio)-pentan-2,3-dion (Reinheitsgrad 90 %) erhalten. IR-, NMR- und Massenspektren der Verbindung stimmen mit der angegebenen Struktur überein.

### Beispiel 3

1,0 g (8,6 mmol) Thiophen-2-thiol werden in 8 ml Ether gelöst. Unter Kühlen auf 0°C werden 1,03 g (9,1 mmol) Kalium-tert.-butylat zugesetzt. Nach beendeter Zugabe wird für weitere 15 Minuten bei 0°C nachgerührt; dann werden 1,5 g (9,5 mmol) 1-Brombutan-2,3-dion zugesetzt. Man läßt den Ansatz auf Raumtemperatur erwärmen und rührt 4 Stunden nach; dann wird das Gemisch auf Wasser gegeben und dreimal mit Ether extrahiert. Die vereinigten organischen Phasen werden über Natriumsulfat getrocknet.

Nach dem Abziehen des Lösungsmittels verbleiben 720 mg eines braunen Öls.

Zur weiteren Reinigung wird chromatographiert. Laufmittel ist dabei zunächst n-Hexan, später wird dann mit einem Gemisch von n-Hexan/Methyl-tert.-butylether (10:1 Volumenteile) eluiert. Es werden 0,2 g 4-(2-Thienylthio)-butan-2,3-dion (Reinheitsgrad 90 %) erhalten. IR-, NMR- und Massenspektren der Verbindung stimmen mit der angenommenen Struktur überein.

## Patentansprüche

1. Verwendung von Verbindungen der Formel 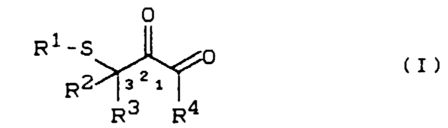 als Aromastoffe
worin
R¹ C₁-C₁₂-Alkyl, C₂-C₁₂-Alkenyl, C₂-C₁₂-Alkinyl, C₃-C₁₂-Cycloalkyl, C₆-C₁₂-Ar-C₁-C₆-alkyl, gegebenenfalls substituiertes C₆-C₁₂-Aryl, einen 5- oder 6-gliedrigen Heterocyclus mit Sauerstoff, Schwefel und/oder Stickstoff als Heteroatom und mit 0 bis 3 Doppelbindungen, der durch 1 bis 3 C₁-C₃-Alkylgruppen substituiert sein kann, oder Heterocyclo-methyl,
R² Wasserstoff oder C₁-C₄-Alkyl,
R³, R⁴ unabhängig voneinander Wasserstoff, C₁-C₈-Alkyl, C₂-C₈-Alkenyl, C₂-C₈-Alkinyl, C₅-C₁₂-Cycloalkyl, C₆-C₁₂-Aryl, oder
R² und R³ gemeinsam oder
R³ und R⁴ gemeinsam einen zweiwertigen Rest, der mit dem C-Atom 3 beziehungsweise mit den C-Atomen 1, 2 und 3 einen 5- bis 7-gliedrigen Ring enthaltend 0 bis 2 Heteroatome aus der Reihe Stickstoff, Sauerstoff, Schwefel bildet,
bedeuten.

2. Verbindungen der Formel (I) gemäß Anspruch 1, ausgenommen
1-Phenylthio -2.3-pentandion,
1-Phenylthio -2.3-butandion,
Ethylthiomethylglyoxal,
(2-Hydroxyethyl)-thio-methylglyoxal und
1-Phenyl-3-methylthio-1.2-butandion.

3. Verfahren zur Herstellung der Verbindungen nach Anspruch 2 durch
a) Umsetzung von Thiolen der Formel
R¹-SH (II)
mit Ketonen der Formel 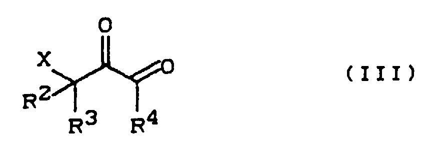 worin
X ein Halogenatom, p-Toluolsulfonat, Methansulfonat, Acetyl oder Benzoyl bezeichnet,
b) Umsetzung von Methylthioethern der Formel
R¹-S-CH₃ (IV)
mit den Acetalen von α-Ketocarbonsäureestern der Formel 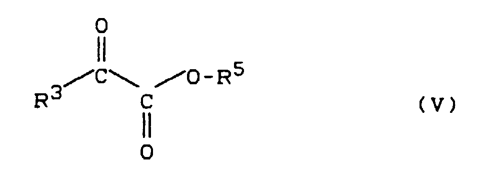 worin R⁵ für Methyl, Ethyl, n-Propyl oder Isopropyl steht,
in Gegenwart starker Basen (zur Deprotonierung) und anschließende Acetalspaltung oder
c) Umsetzung von Thioalkandionen der Formel 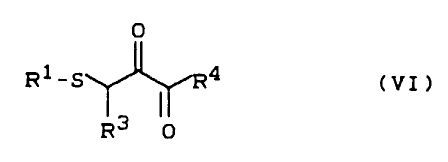 mit Verbindungen der Formel
R²-X (VII)
worin X die unter a) angegebene Bedeutung besitzt, in Gegenwart von Deprotonierungsmitteln,
wobei die Substituenten R¹ bis R⁴ jeweils die für die Verbindungen (I) angegebenen Bedeutungen besitzen mit der Ausnahme, daß R¹ in Formel (IV) nicht für Wasserstoff steht.

## Claims

1. Use of compounds of the formula 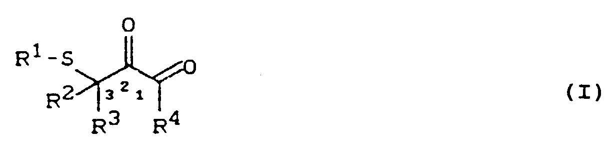 as aroma substances
wherein
R¹ denotes C₁-C₁₂-alkyl, C₂-C₁₂-alkenyl, C₂-C₁₂-alkinyl, C₃-C₁₂-cycloalkyl, C₆-C₁₂-ar-C₁-C₆-alkyl, optionally substituted C₆-C₁₂-aryl, a 5- or 6-membered heterocyclic radical with oxygen, sulphur and/or nitrogen as the heteroatom and with 0 to 3 double bonds, which can be substituted by 1 to 3 C₁-C₃-alkyl groups, or heterocyclo-methyl,
R² denotes hydrogen or C₁-C₄-alkyl and
R³ and R⁴ independently of one another denote hydrogen, C₁-C₈-alkyl, C₂-C₈-alkenyl, C₂-C₈-alkinyl, C₅-C₁₂-cycloalkyl or C₆-C₁₂-aryl or
R² and R³ together or
R³ and R⁴ together denote a divalent radical which, with the C atom 3 or with the C atoms 1, 2 and 3, forms a 5- to 7-membered ring containing 0 to 2 heteroatoms from the series consisting of nitrogen, oxygen and sulphur.

2. Compounds of the formula (I) according to Claim 1, excluding
1-phenylthio-2,3-pentanedione,
1-phenylthio-2,3-butanedione,
ethylthiomethylglyoxal,
(2-hydroxyethyl)-thio-methylglyoxal and
1-phenyl-3-methylthio-1,2-butanedione.

3. Process for the preparation of compounds according to Claim 2, by
a) reaction of thiols of the formula
R¹-SH (II)
with ketones of the formula 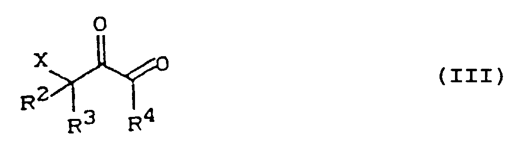 wherein
X designates a halogen atom, p-toluenesulphonate, methanesulphonate, acetyl or benzoyl,
b) reaction of methyl thioethers of the formula
R¹-S-CH₃ (IV)
with the acetals of α-ketocarboxylic acid esters of the formula 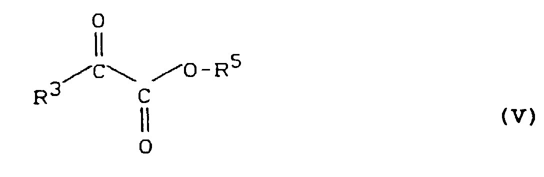 wherein R⁵ represents methyl, ethyl, n-propyl, or isopropyl,
in the presence of strong bases (for deprotonation), and subsequent acetal cleavage or
c) reaction of thioalkanediones of the formula 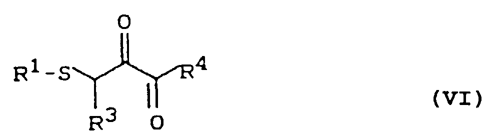 with compounds of the formula
R²-X (VII)
wherein X has the meaning given under a), in the presence of deprotonating agents,
wherein the substituents R¹ to R⁴ in each case have the meanings given for the compounds (I) with the exception that R¹ in formula (IV) does not represent hydrogen.

## Revendications

1. Utilisation en tant que matières aromatiques des composés de formule 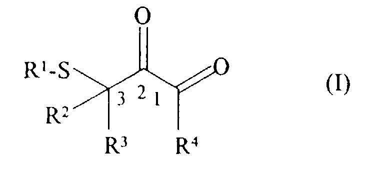 dans laquelle
R¹ représente un groupe alkyle en C₁-C₁₂, alcényle en C₂-C₁₂, alcynyle en C₂-C₁₂, cycloalkyle en C₃-C₁₂, (aryle en C₆-C₁₂)-alkyle en C₁-C₆, aryle en C₆-C₁₂ éventuellement substitué, un hétérocycle à 5 ou 6 chaînons contenant de l'oxygène, du soufre et/ou de l'azote en tant qu'hétéroatome et de 0 à 3 doubles liaisons, et qui peut porter un à trois substituants alkyle en C₁-C₃, ou un groupe hétérocyclo-méthyle,
R² représente l'hydrogène ou un groupe alkyle en C₁-C₄,
R³ et R⁴ représentent chacun, indépendamment l'un de l'autre, l'hydrogène, un groupe alkyle en C₁-C₈, alcényle en C₂-C₈, alcynyle en C₂-C₈, cycloalkyle en C₅-C₁₂, aryle en C₆-C₁₂, ou bien
R² et R³, ensemble, ou bien
R³ et R⁴, ensemble, représentent un radical divalent qui forme avec l'atome de carbone 3 et respectivement les atomes de carbone 1, 2 et 3 un cycle de 5 à 7 chaînons contenant 0 à 2 hétéroatomes choisis parmi l'azote, l'oxygène et le soufre.

2. Composés de formule (I) selon revendication 1, à l'exception des suivants :
1-phénylthio-2,3-pentanedione,
1-phénylthio-2,3-butanedione,
éthylthiométhylglyoxal,
(2-hydroxyéthyl)-thiométhylglyoxal et
1-phényl-3-méthylthio-1,2-butanedione

3. Procédé de préparation des composés de la revendication 2
a) par réaction des thiols de formule
R¹-SH (II)
avec des cétones de formule 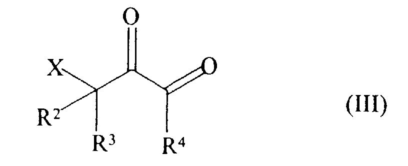 dans laquelle
X représente un atome d'halogène, un groupe p-toluènesulfonate, méthanesulfonate, acétyle ou benzoyle,
b) par réaction des thioéthers méthyliques de formule
R¹-S-CH₃ (IV)
avec les acétals des esters d'acide α-cétocarboxyliques de formule 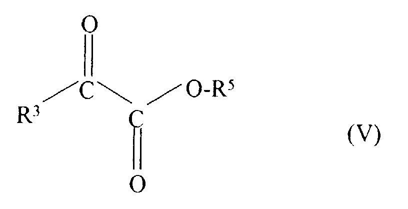 dans laquelle R⁵ représente un groupe méthyle, éthyle, n-propyle ou isopropyle, en présence de bases fortes (pour déprotonation), en faisant suivre de la scission de l'acétal, ou bien
c) par réaction des thioalcanediones de formule 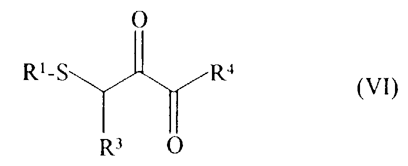 avec des composés de formule
R²-X (VII)
dans laquelle X a les significations indiquées ci-dessus sous a), en présence d'agents déprotonisants,
les symboles R¹ à R⁴ ayant dans tous les cas les significations indiquées en référence aux composés (I), sous réserve que, dans la formule (IV), R¹ ne peut représenter l'hydrogène.
